# EUROPÄISCHE PATENTSCHRIFT

(11) **EP 3 035 839 B1**
(45) Veröffentlichungstag und Bekanntmachung des Hinweises auf die Patenterteilung: **30.10.2019**
(21) Anmeldenummer: 14745080.3
(22) Anmeldetag: 23.07.2014
(51) Int. Cl.: A61B 1/12, B08B 9/032, A61B 90/00

(54) **VERFAHREN UND VORRICHTUNG ZUM REINIGEN EINES CHIRURGISCHEN INSTRUMENTS**
METHOD AND DEVICE FOR CLEANING A SURGICAL INSTRUMENT
PROCÉDÉ ET DISPOSITIF DE NETTOYAGE D'UN INSTRUMENT DE CHIRURGIE

(30) Priorität: 21.08.2013 DE 102013216532
(43) Veröffentlichungstag der Anmeldung: 29.06.2016
(73) Patentinhaber: Olympus Winter & Ibe GmbH, 22045 Hamburg (DE)
(72) Erfinder: AEHLIG, Dennis, 22081 Hamburg (DE)
(74) Vertreter: Seemann & Partner Patentanwälte mbB
(86) Internationale Anmeldenummer: PCT/EP2014/002012
(87) Internationale Veröffentlichungsnummer: WO 2015/024618

(56) Entgegenhaltungen:
- EP-A1- 2 060 276
- EP-A1- 2 283 789
- WO-A1-96/33819
- GB-A- 2 289 512
- US-A1- 2007 102 045

## Beschreibung

Die Erfindung betrifft ein Verfahren zum Reinigen eines wenigstens einen Kanal aufweisenden chirurgischen Instruments, insbesondere Endoskops, mittels eines Reinigungsfluides.

Die Erfindung betrifft des Weiteren eine Vorrichtung zur Reinigung eines wenigstens einen Kanal aufweisenden chirurgischen Instruments, insbesondere Endoskops, mittels eines Reinigungsfluides. Die Erfindung betrifft ferner eine Verwendung.

Im Stand der Technik sind flexible Endoskope mit internen Kanälen bekannt, die beispielsweise in der gastrointestinalen Chirurgie Verwendung finden. Zur Reinigung derartiger Endoskope werden die internen Kanäle in der Regel mit einem Spülmedium unter konstantem Druck bei gleichbleibendem Volumenstrom gespült.

Zum Entfernen von angetrockneten und im Kanal verklemmten Partikeln erfolgt beispielsweise eine aufwendige Vorbehandlung, bei der die Endoskope bzw. die Kanäle manuell gebürstet werden.

Im Hinblick hierauf sind Verfahren entwickelt worden, bei denen alternierend Luft und Wasser durch die Kanäle gepumpt werden. Des Weiteren ist die sogenannte Two-Phase-Flow-Technik bekannt, bei der feinste Flüssigkeitstropfen in einer Gasphase das gleiche Ergebnis wie das manuelle Bürsten erzeugen sollen.

Die bekannten Verfahren zur Vermeidung des manuellen Bürstens haben den Nachteil, dass entsprechende Reinigungsvorrichtungen zusätzliche Bauteile für die Luftversorgung und die Luftwege aufweisen müssen und entsprechend komplexer und fehleranfälliger sind als einfache Spülvorrichtungen.

Des Weiteren sind die genannten Verfahren zur Entfernung von sogenannten Biofilmen, bei denen eine großflächige und zusammenhängende Verschmutzung des Endoskopkanals vorliegt, nur eingeschränkt geeignet. Ein Abtrag solcher Substanzen ist mit den genannten Verfahren nicht oder nur mit sehr langen Verfahrenszeiten realisierbar.

EP 2 060 276 B1 offenbart eine Vorrichtung für das Reinigen von Endoskopen, die eine Gruppe von mehreren Endoskopkanälen umfassen. Es sind Pumpen vorgesehen, wobei in Abhängigkeit eines Druckmesswertes der Reinigungsprozess des Endoskops gesteuert wird.

WO 96/33819 A1 offenbart ein Verfahren und eine Vorrichtung zum Reinigen von Hohlkörpern. Es wird eine Reinigungsflüssigkeit pulsiert durch den Hohlkörper geleitet, wobei gleichzeitig eine Ultraschallwelle auf die Reinigungsflüssigkeit gegeben wird. Zwischen zwei pulsierten Strömen der Reinigungsflüssigkeit kommt die Reinigungsflüssigkeit zum Stillstand.

Ausgehend von diesem Stand der Technik liegt der vorliegenden Erfindung die Aufgabe zugrunde, die Reinigung eines Endoskops mit einem Endoskopkanal zu vereinfachen und insbesondere die Entfernung eines Biofilmes von einer Innenseite des Endoskopkanals zu verbessern.

Diese Aufgabe wird gemäß der Erfindung gelöst durch ein Verfahren nach Anspruch 1 zum Reinigen eines wenigstens einen Kanal aufweisenden chirurgischen Instruments, insbesondere Endoskops, wobei der Kanal aus einem flexiblen Material ist, mittels eines Reinigungsfluides umfassend die folgenden Verfahrensschritte:
- Beaufschlagen des Reinigungsfluides mit einem Druck,
- Spülen des Kanals mit dem Reinigungsfluid und
- Variieren des Drucks während des Spülens, so dass eine Deformation des Kanals bewirkt wird, wobei durch die Deformation des Kanals an der Innenwand des Kanals anhaftende Verschmutzungen, nämlich ein Biofilm, abgelöst und ausgespült werden, wobei der Druck während des Spülens in regelmäßigen zeitlichen Abständen wiederholt variiert wird, wobei das Beaufschlagen des Reinigungsfluids mit dem Druck unter Verwendung einer getakteten Pumpvorrichtung, insbesondere einer Membranpumpe, erfolgt.

Das Variieren des auf das Reinigungsfluid ausgeübten Drucks oder die Druckvariation in dem Reinigungsfluid, insbesondere im Kanal, insbesondere im Endoskopkanal, bewirkt insbesondere Deformationen des Kanals, wodurch sich der Kanal bei Erhöhung des Drucks weitet und bei Verringerung des Drucks verengt. Durch diese Deformationen werden an der Innenwand des Kanals anhaftende Verschmutzungen gelöst und können dadurch leichter mittels des Reinigungsfluides aus dem Kanal gespült werden.

Ein besonderer Vorteil des erfindungsgemäßen Verfahrens besteht darin, dass auch Biofilme und andere großflächige Verschmutzungen abgelöst und ausgespült werden.

Insgesamt wird dadurch das Reinigungsergebnis verbessert und die Dauer der Reinigung verkürzt.

Ein weiterer Vorteil der kürzeren Reinigungsdauer ist außerdem, dass weniger Reinigungsflüssigkeit verbraucht wird. Dadurch wird die Reinigung kostengünstiger und umweltfreundlicher. Erfindungsgemäß wird der Druck während des Spülens wiederholt variiert, wodurch die Reinigungswirkung weiter verbessert wird.

Des Weiteren wird der Druck in regelmäßigen zeitlichen Abständen wiederholt variiert. Darunter wird im Rahmen der Erfindung insbesondere ein periodisches, zyklisches oder oszillierendes Variieren des Drucks verstanden. Dieses bietet den besonderen Vorteil einer technisch einfachen Umsetzung, beispielsweise basierend auf rotierenden Antriebsmaschinen mit entsprechenden Druckaktuatoren.

Erfindungsgemäß erfolgt das Beaufschlagen des Reinigungsfluides mit dem Druck unter Verwendung einer getakteten Pumpvorrichtung. Hierunter wird im Rahmen der Erfindung insbesondere eine Pumpvorrichtung oder eine Fördervorrichtung verstanden, die konstruktionsbedingt mehrere Arbeitstakte oder Arbeitsphasen aufweist, beispielsweise einen Ansaugtakt und einen Verdichtungstakt oder Ausstoßtakt. Dadurch ist unmittelbar ein pulsierendes oder oszillierendes Druckniveau in dem geförderten oder gepumpten Reinigungsfluid erzeugbar. Eine derartige getaktete Pumpvorrichtung ist beispielsweise eine Hubkolbenpumpe oder eine Membranpumpe.

Eine Membranpumpe hat dabei insbesondere den weiteren Vorteil, dass sie keine Schleifdichtung aufweist, wodurch eine entsprechende getaktete Pumpvorrichtung auch zum Fördern von verunreinigtem Reinigungsfluid geeignet ist.

Im Rahmen der Erfindung geeignete Membranpumpen werden beispielsweise von der Firma KNF FLODOS AG, Sursee, Schweiz, unter der Produktbezeichnung NF 600 oder NF 1.600 sowie von der Firma Eckerle Industrie-Elektronik GmbH, Malsch, Bundesrepublik Deutschland, unter der Produktbezeichnung CDP 6800, CDP 8800, DDP 5800 oder DDP 550 angeboten.

Eine besonders bevorzugte Weiterbildung des erfindungsgemäßen Verfahrens ist dadurch ausgezeichnet, dass als weiterer Verfahrensschritt eine Reinigungskontrolle des Kanals umfasst ist.

Bei der Reinigungskontrolle handelt es sich insbesondere um eine Prüfung, inwieweit die Reinigung erfolgreich war oder fortgesetzt oder wiederholt werden muss. Die Prüfung erfolgt beispielsweise mittels einer Bestimmung einer Durchflussrate und/oder mittels einer Bestimmung eines Druckabfalls in dem Kanal.

Vorzugsweise wird das Reinigungsfluid während der Reinigungskontrolle mit einem konstanten Druck beaufschlagt. Hierdurch lassen sich in der Regel genauere Messergebnisse oder Prüfresultate erreichen.

Das erfindungsgemäße Verfahren ist insbesondere auch zur Reinigung von Endoskopzubehör und Zubehör für Endoskop-Reinigungsapparate, beispielsweise Schläuche und dergleichen, geeignet.

Die der Erfindung zugrunde liegende Aufgabe wird des Weiteren gelöst durch eine Vorrichtung gemäß Anspruch 3 zur Reinigung eines wenigstens einen Kanal aufweisenden chirurgischen Instruments, insbesondere Endoskops, mittels eines Reinigungsfluides umfassend eine Pumpeinrichtung für das Reinigungsfluid und einen Anschluss für den Kanal zur Spülung des Kanals mit dem Reinigungsfluid, wobei der Kanal aus einem flexiblen Material ist, wobei die Pumpeinrichtung dazu ausgebildet ist, das Reinigungsfluid während einer Spülung des Kanals mit einem variierenden Druck zu beaufschlagen, wobei die Pumpeinrichtung wenigstens zwei Pumpvorrichtungen umfasst, wobei eine der Pumpvorrichtungen als getaktete Pumpvorrichtung und eine andere der Pumpvorrichtungen als Dauerpumpvorrichtung ausgebildet sind, wobei eine Druckvariation in dem Reinigungsfluid bewirkt wird, die eine Deformation des Kanals bewirkt, so dass an der Innenwand des Kanals anhaftende Verschmutzungen, nämlich ein Biofilm, abgelöst und ausgespült werden.

Zum Beaufschlagen des Reinigungsfluides mit einem variierenden Druck umfasst die Pumpeinrichtung einer erfindungsgemäßen Reinigungsvorrichtung eine getaktete Pumpvorrichtung, beispielsweise eine Hubkolbenpumpe oder eine Membranpumpe.

Der zu reinigende Kanal ist aus einem flexiblen Material. Vorzugsweise ist das Endoskop ein flexibles Endoskop.

Die Pumpeinrichtung umfasst eine Dauerpumpvorrichtung und eine, insbesondere automatische, Steuerungsvorrichtung zur Veränderung eines Ausgangsdrucks der Dauerpumpvorrichtung.

Unter einer Dauerpumpvorrichtung wird insbesondere eine Pumpvorrichtung oder Fördervorrichtung mit kontinuierlicher, insbesondere ungetakteter, Förderung verstanden. Ein Variieren oder eine Variation des Drucks oder Ausgangsdrucks der Dauerpumpvorrichtung wird dabei insbesondere durch Variation der Pump- oder Förderleistung mittels der Steuerungsvorrichtung erreicht. Dies geschieht vorzugsweise automatisch.

Eine geeignete Dauerpumpvorrichtung ist beispielsweise eine Kreiselpumpe mit wechselstrombetriebenem Elektroantrieb, wobei die Steuerungsvorrichtung beispielsweise einen dem Elektroantrieb vorgeschalteten Frequenzumrichter für den Wechselstrom umfasst. Durch Änderung der Frequenz des Wechselstroms ist in diesem Fall die Drehzahl der Kreiselpumpe und entsprechend deren Förderleistung und somit der Ausgangsdruck variierbar.

Erfindungsgemäß umfasst die Pumpeinrichtung wenigstens zwei Pumpvorrichtungen, wobei eine der Pumpvorrichtungen als getaktete Pumpvorrichtung und eine andere der Pumpvorrichtungen als Dauerpumpvorrichtung ausgebildet sind.

Dadurch werden die Vorteile einer Dauerpumpvorrichtung, insbesondere eine hohe Fördermenge pro Zeiteinheit, kombiniert mit den Vorteilen einer getakteten Pumpvorrichtung, insbesondere einer konstruktionsbedingten Variation des Ausgangsdrucks synergetisch kombiniert.

Des Weiteren ist vorteilhaft, dass die Fördermenge des Reinigungsfluides sowie die Variation des Drucks innerhalb des Reinigungsfluides, insbesondere die Amplitude und die Frequenz der Druckvariation, unabhängig voneinander vorgegeben werden können, indem die jeweilige Förderleistung der Dauerpumpvorrichtung einerseits und der getakteten Pumpvorrichtung andererseits geeignet eingestellt werden.

Vorzugsweise umfasst die erfindungsgemäße Vorrichtung eine Anschlussgruppe mit mehreren Anschlüssen für jeweils einen Kanal, insbesondere Endoskopkanal.

Dadurch wird die Dauer der Reinigung eines chirurgischen Instruments weiter verringert, weil mehrere Kanäle des chirurgischen Instruments gleichzeitig gereinigt werden können.

Es ist des Weiteren vorteilhaft, wenn die Vorrichtung mehrere Anschlüsse oder Anschlussgruppen umfasst, wobei die Pumpeinrichtung wenigstens eine Pumpvorrichtung umfasst, die einem Anschluss oder einer Anschlussgruppe ausschließlich zugeordnet ist.

Hierdurch wird eine hohe Flexibilität beim Betrieb der erfindungsgemäßen Vorrichtung erreicht, weil mittels der einen, einem Anschluss oder einer Anschlussgruppe ausschließlich zugeordneten Pumpvorrichtung das Druckniveau dieses Anschlusses bzw. dieser Anschlussgruppe unabhängig von anderen Anschlüssen und Anschlussgruppen vorgebbar ist.

Eine vorteilhafte Weiterbildung der erfindungsgemäßen Vorrichtung ist ferner dadurch ausgezeichnet, dass ein Anschluss oder eine Anschlussgruppe eine Kontrollvorrichtung zur Reinigungskontrolle von an den Anschluss oder die Anschlussgruppe anschließbaren Kanälen, insbesondere mittels einer Bestimmung einer Durchflussrate und/oder mittels einer Bestimmung eines Druckabfalls in dem Kanal, umfasst.

Hierfür weist die Kontrollvorrichtung beispielsweise einen Drucksensor zur Bestimmung eines Drucks des Reinigungsfluides und/oder einen Durchflusssensor zur Bestimmung einer Durchflussmenge oder einer Durchflussrate auf.

Die der Erfindung zugrunde liegende Aufgabe wird ferner gelöst durch eine Verwendung einer getakteten Pumpvorrichtung gemäß Anspruch 7, insbesondere einer Membranpumpe, und einer Dauerpumpvorrichtung, insbesondere einer Kreiselpumpe, zum Reinigen eines Kanals eines chirurgischen Instruments, insbesondere eines Endoskopkanals. Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1a:: schematisch das Entfernen eines Schmutzpartikels aus einem Endoskopkanal durch Spülen mit einem Reinigungsfluid;
- Fig. 1b:: schematisch das Entfernen eines Biofilmes aus einem Endoskopkanal durch Spülen mit einem Reinigungsfluid;
- Fig. 2:: schematisch ein Druckprofil eines Reinigungsfluids in einem Endoskopkanal bei einem Reinigungsverfahren gemäß der Erfindung;
- Fig. 3a:: schematisch die Funktionsweise einer Membranpumpe (Ansaugtakt);
- Fig. 3b:: schematisch die Funktionsweise einer Membranpumpe (Ausstoßtakt); und
- Fig. 4:: schematisch ein beispielhaftes Reinigungs- und Desinfektionsgerät gemäß der Erfindung.

In den Zeichnungen sind jeweils gleiche oder gleichartige Elemente und/oder Teile mit denselben Bezugsziffern versehen, so dass von einer erneuten Vorstellung jeweils abgesehen wird.

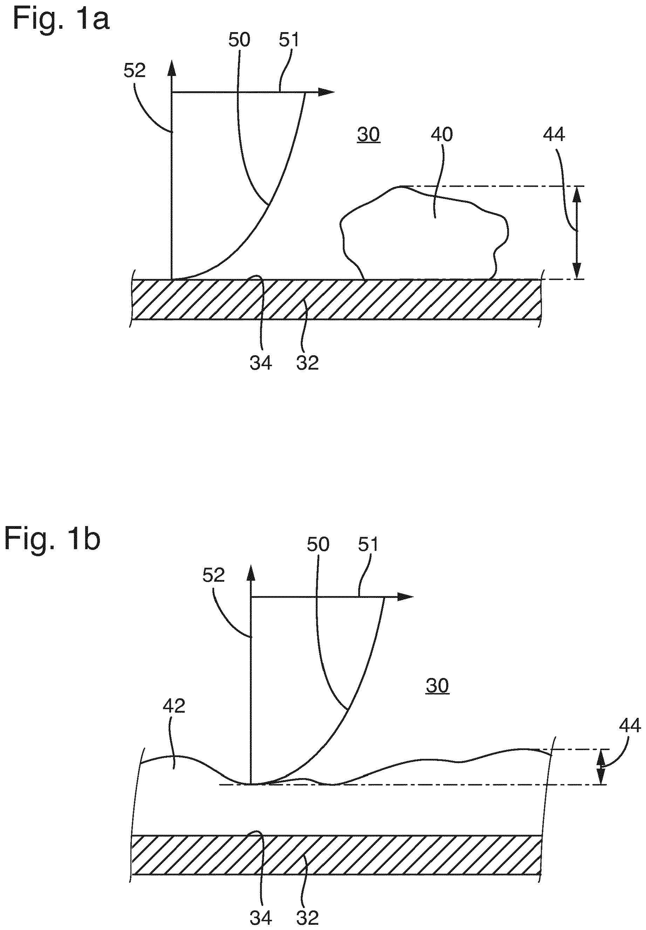

Fig. 1a und 1b zeigen jeweils schematisch einen Ausschnitt eines Endoskopkanals 30, der seitlich begrenzt ist durch eine, insbesondere flexible, Seitenwand 32. Die Seitenwand 32 bzw. der Endoskopkanal 30 weist eine innere Oberfläche 34 auf.

Der Endoskopkanal 30 ist in Umfangsrichtung geschlossen, beispielsweise indem die Seitenwand 32 schlauchförmig ausgebildet ist. Dies ist in Fig. 1a aus Gründen der Übersichtlichkeit nicht dargestellt.

In Fig. 1a haftet an der inneren Oberfläche 34 des Endoskopkanals 30 ein Schmutzpartikel 40 an, das durch Spülen des Endoskopkanals 30 mit einer Reinigungsflüssigkeit entfernt werden soll. Ebenfalls schematisch gezeigt ist ein Strömungsprofil 50 der Reinigungsflüssigkeit, das die Strömungsgeschwindigkeit 51 der Reinigungsflüssigkeit in Abhängigkeit des Abstandes 52 von der inneren Oberfläche 34 an einem willkürlich ausgewählten Punkt in Längsrichtung des Endoskopkanals 30 zu einem willkürlich gewählten Zeitpunkt darstellt.

Das Schmutzpartikel 40 hat eine effektive Angriffsfläche 44, auf die der Strom der Reinigungsflüssigkeit einwirkt. Dadurch wirken Scherkräfte auf das Schmutzpartikel 40.

Insbesondere wenn der Strom der Reinigungsflüssigkeit vor dem Schmutzpartikel 40 an der Oberfläche 34 des Endoskopkanals 30 anliegt wird durch die Scherkräfte eine Grenzschicht zwischen Schmutzpartikel 40 und Oberfläche 34 belastet. Dies führt zum Ablösen des Schmutzpartikels 40 von der Oberfläche 34, wobei das abgelöste Schmutzpartikel 40 mit dem Strom der Reinigungsflüssigkeit fortgetragen und aus dem Endoskopkanal ausgespült wird.

Je höher die Scherkräfte sind, desto schneller und vollständiger erfolgt das Ablösen des Schmutzpartikels 40 von der inneren Oberfläche 34 der Seitenwand 32, wobei die Scherkräfte zusätzlich verstärkt werden, wenn es am Schmutzpartikel 40 zu einer Verwirbelung der Reinigungsflüssigkeit kommt.

In Fig. 1b ist schematisch der Endoskopkanal 30 aus Fig. 1a mit einer Verschmutzung in Form eines Biofilmes 42 gezeigt. Bei dem Biofilm 42 handelt es sich um eine großflächige, geschlossene Schmutzschicht auf der inneren Oberfläche 34 der Seitenwand 32 des Endoskopkanals 30, die beispielsweise eine zähflüssige bis schleimige Konsistenz aufweist.

Der Biofilm 42 weist eine viel geringere effektive Angriffsfläche 44 auf als ein isoliertes Schmutzpartikel 40 und wird im Wesentlichen laminar überströmt. Insbesondere reicht die effektive Angriffsfläche 44 nicht bis zur inneren Oberfläche 34 der Seitenwand 32 und ist auf die oberen, von der Oberfläche 34 entfernten Schichten des Biofilmes 42 begrenzt.

Dadurch wird der Biofilm 42 bei herkömmlichem Spülen mit einer Reinigungsflüssigkeit bei konstantem Druck und Volumenstrom nur oberflächlich angegriffen, bestenfalls schichtweise abgetragen und somit unzureichend oder erst nach sehr langer Spüldauer abgetragen.

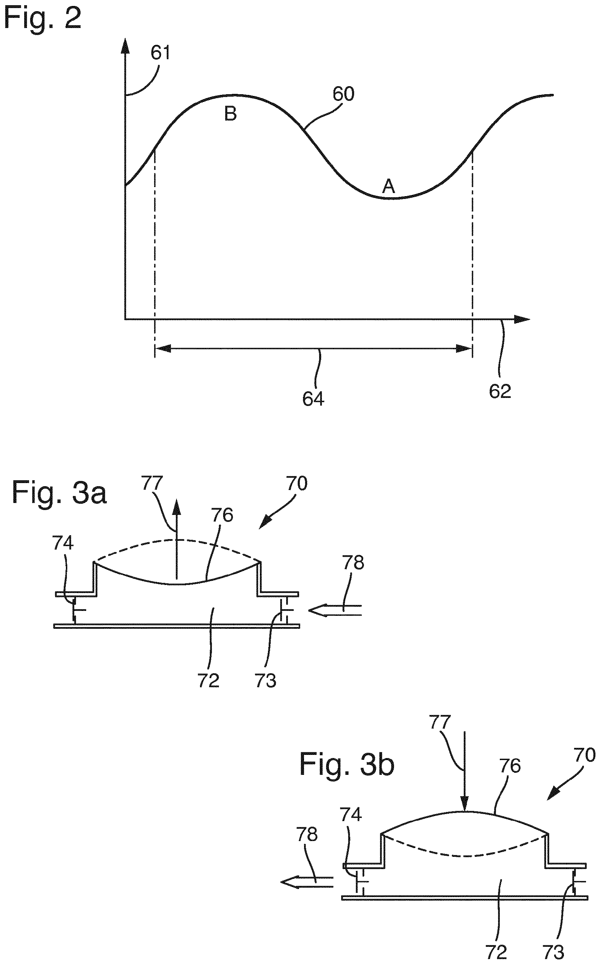

Erfindungsgemäß ist vorgesehen, die Reinigungsflüssigkeit in dem Endoskopkanal 30 mit einem variierenden Druck zu beaufschlagen. Ein Beispiel eines derartigen Druckprofils 60 ist schematisch in Fig. 2 dargestellt. Das Druckprofil 60 zeigt einen Druck 61 der Reinigungsflüssigkeit in dem Endoskopkanal in Abhängigkeit der Zeit 62.

Das Druckprofil 60 weist beispielsweise wiederkehrende Perioden 64 auf, die jeweils eine Niederdruckphase oder einen Niederdrucktakt A und eine Hochdruckphase oder einen Hochdrucktakt B umfassen. Der zeitlich variierende Druck 61 der Reinigungsflüssigkeit in dem Endoskopkanal 30 führt zu elastischen Deformierungen oder Deformationen der, insbesondere flexiblen, Seitenwand 32 des Endoskopkanals 30, wobei sich der Endoskopkanal 30 beim Wechsel einer Niederdruckphase B zu einer Hochdruckphase A weitet und beim Wechseln einer Hochdruckphase B zu einer Niederdruckphase A verengt.

Der Druckunterschied zwischen einer Hochdruckphase und einer Niederdruckphase ist dabei vorzugsweise derart vorgegeben, dass die Strömungsrichtung des Spülmittels im Endoskopkanal 30 beim Wechsel zwischen Hochdruckphase zu Niederdruckphase und umgekehrt jeweils beibehalten wird.

Die wiederholten Deformationen der Seitenwand 32 bewirken ein wesentlich schnelleres und vollständigeres Ablösen des Biofilmes 42 von der Oberfläche 34 des Endoskopkanals 30, als dies alleine aufgrund der mittels der strömenden Reinigungsflüssigkeit auf den Biofilm einwirkenden Scherkräfte möglich wäre.

Dies lässt sich durch beliebige Druckprofile 60 mit zeitlich wechselndem Druck 61, beispielsweise auch durch einzelne Druckstöße in unregelmäßiger zeitlicher Abfolge, erzielen.

Beispielsweise lässt sich ein erfindungsgemäßes Druckprofil 60 mittels einer Membranpumpe 70 realisieren.

In Fig. 3a und 3b ist schematisch die Funktionsweise einer Membranpumpe 70 dargestellt.

Die Membranpumpe 70 umfasst ein Gehäuse mit einer Pumpkammer 72, die einen Einlass mit Einlassventil 73 und einen Auslass mit Auslassventil 74 aufweist. Eine Seitenwand der Pumpkammer 72 ist als flexible oder bewegliche Membran 76 ausgebildet, die von außerhalb der Pumpenkammer 72 mittels eines nicht dargestellten Aktuators bewegt werden kann. Der Aktuator wirkt beispielsweise mechanisch, pneumatisch oder hydraulisch auf die Membran 76.

Fig. 3a zeigt einen ersten Arbeitstakt der Membranpumpe 70, bei dem Reinigungsflüssigkeit in die Pumpkammer 72 angesaugt wird. Hierfür wird die als durchgezogene Linie dargestellte Membran 76 mittels des Aktuators entlang des Pfeils 77 in die gestrichelt dargestellte Position bewegt. Während des Ansaugens oder des Ansaugtaktes ist das Einlassventil geöffnet, um ein Einströmen der Reinigungsflüssigkeit über den Einlass, dargestellt durch den Pfeil 78, zu ermöglichen. Gleichzeitig ist das Auslassventil 74 geschlossen, um einen Rückfluss von Reinigungsflüssigkeit über den Auslass zu verhindern.

Fig. 3b zeigt einen zweiten Arbeitstakt der Membranpumpe 70, bei dem die Reinigungsflüssigkeit in der Pumpkammer 72 über den Auslass unter Druck ausgestoßen wird. Hierfür wird die als durchgezogene Linie dargestellte Membran 76 mittels des Aktuators entlang des Pfeils 77 in die gestrichelt dargestellte Position bewegt. Während des Ausstoßens oder Ausstoßtaktes ist das Auslassventil 74 geöffnet, um ein Ausströmen der Reinigungsflüssigkeit, dargestellt durch den Pfeil 78, zu ermöglichen. Gleichzeitig ist das Einlassventil 73 geschlossen, um einen Rückfluss von Reinigungsflüssigkeit in den Einlass zu verhindern.

Wenn die Reinigungsflüssigkeit am Einlass bereits unter Druck steht oder stromaufwärts des Einlasses mit einem Druck beaufschlagt worden ist, wirkt dieser Druck einem Rückfluss entgegen der Strömungsrichtung 78 beim Ansaugen und Ausstoßen entgegen. In diesem Fall können Einlassventil 73 und/oder Auslassventil 74 gegebenenfalls entfallen.

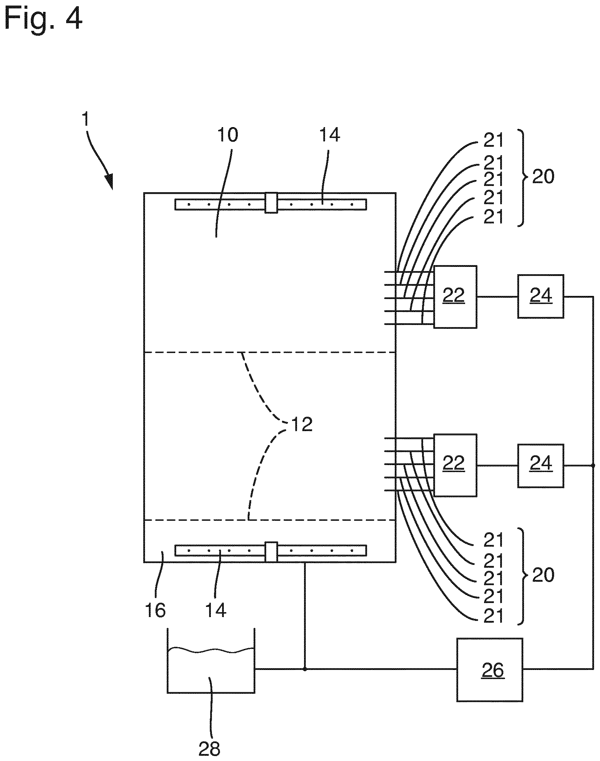

Fig. 4 zeigt ein beispielhaftes Reinigungs- und Desinfektionsgerät 1 gemäß der Erfindung zur Reinigung von Endoskopen. Das Reinigungs- und Desinfektionsgerät 1 umfasst einen Spülraum 10 mit zwei Spülebenen 12, die beispielsweise jeweils einen Drahtkorb, eine Lochplatte oder eine vergleichbare, flüssigkeitsdurchlässige Ablagefläche für ein Endoskop umfassen.

Im Spülraum 10 sind ferner zwei Sprühvorrichtungen 14 zur äußeren Reinigung von auf den Spülebenen 12 abgelegten Endoskopen angeordnet. Die Sprühvorrichtungen 14 weisen geeignete Zuleitungen für, insbesondere flüssige, Reinigungs- und/oder Desinfektionsmittel auf, die aus Gründen der Übersichtlichkeit nicht dargestellt sind.

Zur Reinigung von Endoskopkanälen 30 von auf den Spülebenen 12 abgelegten Endoskopen ist jeder Spülebene 12 eine Anschlussgruppe 20 mit jeweils mehreren Anschlüssen 21 für jeweils einen Endoskopkanal 30 zugeordnet. Jeweils ein Endoskopkanal 30 wird an einen Anschluss 21 angeschlossen und über den Anschluss 21 mit Reinigungsflüssigkeit gespült.

Die dargestellte Anzahl von fünf Anschlüssen 21 pro Anschlussgruppe 20 ist ausdrücklich exemplarisch zu verstehen. Im Rahmen der Erfindungen können auch mehr oder weniger als fünf Anschlüsse 21 pro Anschlussgruppe 20 und/oder eine unterschiedliche Anzahl an Anschlüssen für die beiden Anschlussgruppen 20 vorgesehen sein.

Für jede Anschlussgruppe 20 ist eine Kontrollvorrichtung 22 vorgesehen, die jeweils über eine Anschlussleitung mit Reinigungsflüssigkeit für die Reinigung oder die Spülung der an die Anschlüsse 21 der jeweiligen Anschlussgruppe 20 angeschlossenen Endoskopkanäle 30 versorgt werden.

Die Reinigungsflüssigkeit entstammt beispielsweise einem Vorratsbehälter 28 und gelangt über ein System mit mehreren Pumpen 24, 26 zu den beiden Kontrollvorrichtungen 22.

Die Reinigungsflüssigkeit wird mittels der Kontrollvorrichtungen 22 auf die verschiedenen Anschlüsse 21 der jeweiligen Anschlussgruppe 20 aufgeteilt, wobei beispielsweise einzelne Anschlüsse 21 für den Fall absperrbar ausgebildet sind, dass nur ein Teil der Anschlüsse 21 der betreffenden Anschlussgruppe 20 zur Reinigung eines oder mehrerer Endoskope benötigt werden.

Das Pumpensystem 24, 26 umfasst für jede der Versorgungsvorrichtungen 22 eine als Membranpumpe 70 ausgebildete Boosterpumpe 24 sowie eine gemeinsame, als Kreiselpumpe ausgebildete, Umwälzpumpe 26.

Die Umwälzpumpe 26 wird insbesondere dazu eingesetzt, Reinigungsflüssigkeit durch das beschriebene Leitungssystem und etwaige an die Anschlüsse 21 angeschlossene Endoskopkanäle 30 zu pumpen. Hierfür wird die Reinigungsflüssigkeit insbesondere mittels der Umwälzpumpe 26 mit einem ausreichend hohen, gleichmäßigen Druck beaufschlagt.

Die Boosterpumpen 24 werden im Laufe der Reinigung entsprechend dem erfindungsgemäßen Verfahren zugeschaltet, um den von der Umwälzpumpe 26 bereitgestellten Förderdruck zu variieren oder zu modulieren. Dabei ist die Variation oder Modulation des Druckniveaus für jede der Anschlussgruppen 20 separat zuschaltbar und vorgebbar, da jeder Anschlussgruppe 20 eine eigene Boosterpumpe 24 zugeordnet ist.

Die über die Anschlüsse 21 in einen Endoskopkanal geleitete Reinigungsflüssigkeit durchströmt den Endoskopkanal und gelangt an dessen offenen, dem Anschluss 21 entgegengesetzten Ende in den Spülraum 10. Dort fließt oder tropft die Reinigungsflüssigkeit unter Einfluss der Schwerkraft durch die durchlässigen Spülebenen 12 hindurch in den unteren Bereich des Spülraums 10, der beispielsweise als Sammelbecken 16 ausgebildet ist. Aus dem Sammelbecken 16 wird die Reinigungsflüssigkeit beispielsweise abgelassen und entsorgt oder, wie exemplarisch in Fig. 4 gezeigt, in einem geschlossenen Kreislauf der Umwälzpumpe 26 zur erneuten Verwendung zugeführt.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit insbesondere gekennzeichnet sind, vorzugsweise fakultative Merkmale.

### Bezugszeichenliste

- 1: Reinigungs- und Desinfektionsgerät
- 10: Spülraum
- 12: Spülebene
- 14: Sprühvorrichtung
- 16: Sammelbecken
- 20: Anschlussgruppe
- 21: Anschluss
- 22: Kontrollvorrichtung
- 24: Boosterpumpe
- 26: Umwälzpumpe
- 28: Vorratsbehälter
- 30: Endoskopkanal
- 32: Seitenwand
- 34: innere Oberfläche
- 40: Schmutzpartikel
- 42: Biofilm
- 44: effektive Angriffsfläche
- 50: Strömungsprofil
- 51: Fließgeschwindigkeit
- 52: Abstand
- 60: Druckprofil
- 62: Druck
- 63: Zeit
- 64: Periode
- 70: Membranpumpe
- 72: Pumpkammer
- 73: Einlassventil
- 74: Auslassventil
- 76: Membran
- 77: Membranbewegung
- 78: Förderstrom
- A: Niederdrucktakt
- B: Hochdrucktakt

## Patentansprüche

1. Verfahren zum Reinigen eines wenigstens einen Kanal aufweisenden chirurgischen Instruments, insbesondere Endoskops, wobei der Kanal aus einem flexiblen Material ist, mittels eines Reinigungsfluides umfassend die folgenden Verfahrensschritte:
- Beaufschlagen des Reinigungsfluides mit einem Druck (61),
- Spülen des Kanals (30) mit dem Reinigungsfluid und
- Variieren des Drucks (61) während des Spülens, so dass eine Deformation des Kanals (30) bewirkt wird, wobei durch die Deformation des Kanals (30) an der Innenwand des Kanals (30) anhaftende Verschmutzungen, nämlich ein Biofilm, abgelöst und ausgespült werden, wobei der Druck (61) während des Spülens in regelmäßigen zeitlichen Abständen wiederholt variiert wird, wobei das Beaufschlagen des Reinigungsfluides mit dem Druck (61) unter Verwendung einer Pumpeinrichtung (24; 26; 70) erfolgt, die wenigstens zwei Pumpvorrichtungen (24; 26; 70) umfasst, wobei eine der Pumpvorrichtungen (24; 26; 70) als getaktete Pumpvorrichtung (24; 70), insbesondere als Membranpumpe (70), und eine andere der Pumpvorrichtungen (24; 26; 70) als Dauerpumpvorrichtung (26) ausgebildet sind.

2. Verfahren nach Anspruch 1, **dadurch gekennzeichnet, dass** als weiterer Verfahrensschritt eine Reinigungskontrolle des Kanals (30), insbesondere mittels einer Bestimmung einer Durchflussrate und/oder mittels einer Bestimmung eines Druckabfalls in dem Kanal (30), umfasst ist, wobei insbesondere das Reinigungsfluid während der Reinigungskontrolle mit einem konstanten Druck (61) beaufschlagt wird.

3. Vorrichtung (1) zur Reinigung eines wenigstens einen Kanal aufweisenden chirurgischen Instruments mittels eines Reinigungsfluides umfassend eine Pumpeinrichtung (24, 26; 70) für das Reinigungsfluid und einen Anschluss (21) für den Kanal (30) zur Spülung des Kanals (30) mit dem Reinigungsfluid, wobei der Kanal aus einem flexiblen Material ist, wobei die Pumpeinrichtung (24, 26; 70) dazu ausgebildet ist, das Reinigungsfluid während einer Spülung des Kanals (30) mit einem variierenden Druck zu beaufschlagen, wobei der Druck während des Spülens in regelmäßigen zeitlichen Abständen wiederholt variiert wird, wobei die Pumpeinrichtung (24, 26; 70) wenigstens zwei Pumpvorrichtungen (24; 26; 70) umfasst, wobei eine der Pumpvorrichtungen (24; 26; 70) als getaktete Pumpvorrichtung (24; 70), insbesondere als Membranpumpe (70), und eine andere der Pumpvorrichtungen (24; 26; 70) als Dauerpumpvorrichtung (26) ausgebildet sind, wobei eine Druckvariation in dem Reinigungsfluid bewirkt wird, die eine Deformation des Kanals (30) bewirkt, so dass an der Innenwand des Kanals (30) anhaftende Verschmutzungen, nämlich ein Biofilm, abgelöst und ausgespült werden.

4. Vorrichtung (1) nach Anspruch 3, **dadurch gekennzeichnet, dass** die Vorrichtung (1) eine Anschlussgruppe (20) mit mehreren Anschlüssen (21) für jeweils einen Kanal (30) umfasst.

5. Vorrichtung (1) nach Anspruch 3 oder 4, **dadurch gekennzeichnet, dass** die Vorrichtung (1) mehrere Anschlüsse (21) oder Anschlussgruppen (20) umfasst, wobei die Pumpeinrichtung (24, 26; 70) wenigstens eine Pumpvorrichtung (24; 26; 70) umfasst, die einem Anschluss (21) oder einer Anschlussgruppe (20) ausschließlich zugeordnet ist.

6. Vorrichtung (1) nach einem der Ansprüche 3 bis 5, **dadurch gekennzeichnet, dass** ein Anschluss (21) oder eine Anschlussgruppe (20) eine Kontrollvorrichtung (22) zur Reinigungskontrolle von an den Anschluss (21) oder die Anschlussgruppe (20) anschließbaren Kanälen (30), insbesondere mittels einer Bestimmung einer Durchflussrate und/oder mittels einer Bestimmung eines Druckabfalls in dem Kanal (30), umfasst.

7. Verwendung einer getakteten Pumpvorrichtung (24; 70), insbesondere einer Membranpumpe (70), und einer Dauerpumpvorrichtung (26), insbesondere einer Kreiselpumpe, zum Spülen eines aus einem flexiblen Material bestehenden Kanals (30) eines chirurgischen Instruments, wobei der Druck während des Spülens in regelmäßigen zeitlichen Abständen wiederholt variiert wird, und eine Druckvariation in einem durch die getaktete Pumpvorrichtung (24; 70) und die Dauerpumpvorrichtung (26) mit Druck beaufschlagten Reinigungsfluids bewirkt wird, die eine Deformation des Kanals (30) bewirkt, so dass an der Innenwand des Kanals (30) anhaftende Verschmutzungen, nämlich ein Biofilm, abgelöst und ausgespült werden.

## Claims

1. A method for cleaning, by means of a cleaning fluid, a surgical instrument having at least one channel, in particular an endoscope, wherein the channel is of a flexible material, comprising the following procedural steps:
- Apply pressure (61) to the cleaning fluid,
- Flush the channel (30) with the cleaning fluid, and
- Vary the pressure (61) while flushing to cause a deformation of the channel (30), wherein contaminants, i.e., a biofilm, adhering to the inner wall of the channel (30) are released and rinsed out by the deformation of the channel (30), wherein the pressure (61) is repeatedly varied at regular intervals over time during flushing, wherein cleaning fluid is subject to pressure (61) using a pump device (24; 26; 70) that comprises at least two pump apparatuses (24; 26; 70), wherein one of the pump apparatuses (24; 26; 70) is designed as a cyclical pump apparatus (24; 70), in particular as a diaphragm pump (70), and another of the pump apparatuses (24; 26; 70) is designed as a continuous pump apparatus (26).

2. The method according to claim 1, **characterized in that** an additional procedural step comprises a cleaning check of the channel (30), in particular by means of determining a flow rate and/or by determining a pressure drop within the channel (30), wherein in particular a constant pressure (61) is applied to the cleaning fluid during the cleaning check.

3. An apparatus (1) for cleaning a surgical instrument having at least one channel by means of a cleaning fluid, comprising a pump device (24, 26; 70) for the cleaning fluid and a connection (21) for the channel (30) for flushing the channel (30) with the cleaning fluid, wherein the channel is of a flexible material, wherein the pump device (24, 26; 70) is designed to apply a varying pressure to the cleaning fluid while flushing the channel (30), wherein the pressure is repeatedly varied at regular intervals over time during flushing, wherein the pump device (24, 26; 70) comprises at least two pump apparatuses (24; 26; 70), wherein one of the pump apparatuses (24; 26; 70) is designed as a cyclical pump apparatus (24; 70), in particular as diaphragm pump (70) and another of the pump apparatuses (24; 26; 70) is designed as a continuous pump apparatus (26), wherein a pressure variation in the cleaning fluid is caused that causes a deformation of the channel (30) so that contaminants, i.e., a biofilm, adhering to the inner wall of the channel (30) are released and rinsed out.

4. The apparatus (1) according to claim 3, **characterized in that** the apparatus (1) comprises a connection group (20) with a plurality of connections (21), each for one channel (30).

5. The apparatus (1) according to claim 3 of 4, **characterized in that** the device (1) comprises a plurality of connections (21) or connection groups (20), wherein the pump device (24, 26; 70) comprises at least one pump apparatus (24; 26; 70) to which it is assigned to exclusively one connection (21) or connection group (20).

6. The apparatus (1) according to one of claims 3 to 5, **characterized in that** a connection (21) or connection group (20) comprises a control apparatus (22) for checking the cleaning of channels (30) connectable to the connection (21) or connection group (20), in particular by means of determining a flow rate and/or by means of determining a pressure drop in the channel (30).

7. A use of a cyclical pump apparatus (24; 70), in particular a diaphragm pump (70), and a continuous pump apparatus (26), in particular a reciprocating pump, for cleaning a channel (30) consisting of a flexible material of a surgical instrument, wherein the pressure is repeatedly varied at regular intervals over time during flushing, and a pressure variation is caused in a cleaning fluid to which pressure is applied by the cyclical pump apparatus (24; 70) and the continuous pump apparatus (26) that causes a deformation of the channel (30) so that contaminants, i.e., a biofilm, adhering to the inner wall of the channel (30) are released and rinsed out.

## Revendications

1. Procédé de nettoyage d'un instrument chirurgical, en particulier un endoscope, comportant au moins un conduit constitué d'un matériau souple, au moyen d'un fluide de nettoyage, le procédé comprenant les étapes suivantes :
- application d'une pression (61) au fluide de nettoyage,
- rinçage du conduit (30) avec le fluide de nettoyage et
- faire varier la pression (61) pendant le rinçage de manière à provoquer une déformation du conduit (30), de sorte que des impuretés adhérant à la paroi interne du conduit (30), à savoir un biofilm, soient détachées par la déformation du conduit (30) et évacuées par rinçage, la pression (61) étant modifiée de manière répétée à intervalles de temps réguliers pendant le rinçage, la pression (61) étant appliquée au fluide de nettoyage en utilisant un dispositif de pompage (24 ; 26 ; 70) comprenant au moins deux dispositifs de pompage (24 ; 26 ; 70), l'un des dispositifs de pompage (24 ; 26 ; 70) étant conçu comme un dispositif de pompage (24 ; 70) cadencé, en particulier sous la forme d'une pompe à membrane (70), et un autre des dispositifs de pompage (24 ; 26 ; 70) étant conçu comme un dispositif de pompage (26) en continu.

2. Procédé selon la revendication 1, **caractérisé en ce qu'**il comprend une étape supplémentaire de commande de nettoyage du conduit (30), en particulier au moyen d'une détermination d'un débit et/ou d'une chute de pression dans le conduit (30), le fluide de nettoyage étant en particulier soumis à une pression (61) constante pendant la commande de nettoyage.

3. Dispositif (1) destiné à nettoyer, au moyen d'un fluide de nettoyage, un instrument chirurgical comportant au moins un conduit, lequel dispositif comprend un dispositif de pompage (24, 26 ; 70) pour le fluide de nettoyage et un raccord (21) pour le conduit (30) afin de rincer le conduit (30) avec le fluide de nettoyage, le conduit étant en un matériau souple, le dispositif de pompage (24, 26 ; 70) étant adapté pour appliquer une pression variable au fluide de nettoyage pendant le rinçage du conduit (30), la pression appliquée pendant le rinçage étant modifiée de manière répétée à intervalles de temps réguliers,
le dispositif de pompage (24, 26 ; 70) comprenant au moins deux dispositifs de pompage (24 ; 26 ; 70), l'un des dispositifs de pompage (24 ; 26 ; 70) étant un dispositif de pompage (24 ; 70) cadencé, en particulier étant sous la forme d'une pompe à membrane (70), et un autre des dispositifs de pompage (24 ; 26 ; 70) étant un dispositif de pompage (26) en continu, une variation de pression étant créée dans le fluide de nettoyage, qui provoque une déformation du conduit (30), de sorte que des impuretés adhérant à la paroi interne du conduit (30), à savoir un biofilm, se détachent et s'évacuent par le rinçage.

4. Dispositif (1) selon la revendication 3, **caractérisé en ce que** le dispositif (1) comprend un groupe de raccords (20) ayant une pluralité de raccords (21) pour un conduit (30) respectif.

5. Dispositif (1) selon la revendication 3 ou la revendication 4, **caractérisé en ce que** le dispositif (1) comprend une pluralité de raccords (21) ou de groupes de raccords (20), le dispositif de pompage (24, 26 ; 70) comprenant au moins un dispositif de pompage (24 ; 26 ; 70) qui est exclusivement affecté à un raccord (21) ou un groupe de raccords (20).

6. Dispositif (1) selon l'une quelconque des revendications 3 à 5, **caractérisé en ce qu'**un raccord (21) ou un groupe de raccords (20) comprend un dispositif de commande (22) pour la commande de nettoyage de conduits (30) apte à être relié au raccord (21) ou au groupe de raccords (20), en particulier au moyen de la détermination d'un débit et/ou d'une chute de pression dans le conduit (30).

7. Utilisation d'un dispositif de pompage (24 ; 70) cadencé, en particulier d'une pompe à membrane (70), et d'un dispositif de pompage (26) en continu, en particulier d'une pompe centrifuge, pour le rinçage d'un conduit (30), constitué d'un matériau souple, d'un instrument chirurgical, la pression étant modifiée à intervalles de temps réguliers et répétés pendant le rinçage, et une variation de pression dans un fluide de nettoyage mis sous pression par le dispositif de pompage (24 ; 70) cadencé et le dispositif de pompage (26) en continu étant créée, laquelle variation de pression entraîne une déformation du conduit (30) de telle sorte que des impuretés adhérant à la paroi interne du conduit (30), à savoir un biofilm, sont détachées et s'évacuent par le rinçage.
